# EUROPEAN PATENT APPLICATION

(11) **EP 3 669 922 A1**
(43) Date of publication of application: **24.06.2020**
(21) Application number: 18213011.2
(22) Date of filing: 17.12.2018
(51) Int. Cl.: A61M 21/00, A61M 21/02

(54) **A SYSTEM AND METHOD FOR DELIVERING AN AUDIO OUTPUT**

(71) Applicant: Koninklijke Philips N.V., 5656 AG Eindhoven (NL)
(72) Inventor: VAN DEN ENDE, Daan Anton, 5656 AE Eindhoven (NL); PASTOOR, Sander Theodoor, 5656 AE Eindhoven (NL)
(74) Representative: de Haan, Poul Erik

(57) **Abstract**

A system and method that adapts how an audio output is generated based on a response of a subject's sleep parameters to the audio output. In particular, one or more rules used to generate the audio output are modified in response to how values of sleep parameters (i.e. parameters responsive to a sleep state of the subject) change in response to the audio output. The audio output can be iteratively modified to assess the impact of different audio outputs.

## Description

### FIELD OF THE INVENTION

The present invention relates to the field of systems for providing an audio output to a subject, and in particular to a subject desiring to change their sleep state or encourage certain characteristics of sleep, such as length of deep sleep.

### BACKGROUND OF THE INVENTION

Recent research has shown that auditory stimulation applied during sleep can provide cognitive benefits and enhancements of sleep restoration for a subject or user by at least mitigating disturbances to the subject. It has also been recognized that appropriately controlled audio outputs can help influence a sleep state of the subject, so as to influence at least whether the subject is awake or asleep.

There are numerous different causes for disturbance during sleep, both external to the subject (e.g. traffic noise, aircraft, snoring partners, neighbors, construction noise, insect noise, electrical appliances etc.) and internal. The internal causes include physiological (e.g. tinnitus), psychological (e.g. stress) and behavioral (e.g. poor sleeping practice) causes.

It has been recognized that playing audio can lead to sleep quality improvement, especially by improving the ability to fall asleep in the evening, and also after waking up in the middle of the night. Of course, playing audio can also be exploited to assist in waking the user, for example, to gently move them from a sleep state to an awake state.

The external disturbances can be alleviated by playing a masking sound or by using anti-noise (i.e. using a sound cancellation system). A masking sound is typically a recorded repetitive sound (such as rain or ocean waves) or a generated random waveform with equally distributed acoustic intensity over the audible frequency range (termed 'white noise'). These sounds all aim to drown out sudden and/or annoying external noise and can be clustered under the term 'masking sound'.

Anti-noise (sound cancellation) is a special form of masking sound that requires a microphone close to the ear to pick up the sound vibrations in order to play the right phase-shifted anti-noise. Sleep compatible noise cancellation headphones have been proposed.

The major cause of internal disturbance is typically stress or worrying. This can be mitigated by playing calming sounds or music, guided meditation and/or randomly generated words which all aim to reduce the state of arousal of the mind. These methods all aim to calm the user down so they can go to sleep more easily and can be clustered under the term 'calming audio'. Often, a combination of calming audio and background music is used.

A known sleep-based system uses a sleep detection feature to create a feedback loop. In a particular example, a certain audio output to the subject is stopped in response to the subject falling asleep.

There is a desire to improve systems that provide an audio output for a sleeping subject or a subject attempting to sleep.

### SUMMARY OF THE INVENTION

The invention is defined by the claims.

According to examples in accordance with an aspect of the invention, there is provided a system for delivering an audio output to a subject. The system comprises a sleep parameter monitor adapted to obtain values of one or more sleep parameters of a subject; an audio output device adapted to: determine characteristics for an audio output by processing subject characteristics and/or first values of the one or more sleep parameters using a set of one or more rules; and provide the audio output having the determined characteristics to the subject; and a processing system adapted to modify the set of one or more rules based on second values of the one or more sleep parameters, wherein said second values consist of values, of the one or more sleep parameters, obtained after the audio output device begins providing the audio output to the subject.

The system is adapted to provide an audio output based on characteristics of the subject (e.g. metadata) and/or sleep parameters of the subject. In particular, these characteristics are processed using one or more rules to determine characteristics of the audio output.

A response of a subject to the audio output is then used to modify the rule(s) used to generate the audio output. This provides a feedback system that enables a system to automatically adapt to different users and their response to a particular audio output. Embodiments may thereby provide a system that better assists in changing or encouraging a change in a sleep state of the subject (e.g. encourage a subject to go to sleep or to wake up).

Thus, rather than an audio output being directly adjusted based on a response of the subject's sleep parameters to the audio output (e.g. turning a volume down if an audio output is too high), the rule or rules of how the audio output is generated are modified or calibrated.

Thus, a user-specific and user-adaptable system is provided that adapts to long-term trends of the subject and modifies the underlying methodology of how an audio output is generated for that subject.

Generally speaking, a rule defines a relationship between different inputs and outputs. Some ways to implement rules include IF statements, machine-learning algorithms (i.e. rule-based machine learning), a rule-based system, decision-trees and so on. The set of one or more rules may therefore comprise or consist of a model for generating the characteristics of the audio output.

A sleep parameter is any parameter or characteristic of the subject that is responsive to changes in the sleep state of the subject or other desired sleep-based characteristics (such as amount of slow wave activity or length of (N)REM sleep). Examples of sleep parameters include: temperature, motion, electroencephalogram (EEG) response, neural oscillations, heart rate, breathing rate, sleep onset time and so on. Subject characteristics may include, for example, an identity of the subject, an age of the subject, a gender of the subject and so on. Thus, subject characteristics can comprise metadata of the subject.

Preferably, the processing system is adapted to: determine the response of the one or more sleep parameters to the audio output using the second values of the one or more sleep parameters; and modify the set of one or more rules based on the determined response of the one or more sleep parameters to the audio output. In this way, the set of one or more rules can be modified to reflect how the subject responds to a particular audio output, enabling future audio output of the system to be tailored to a particular subject or user. The second values are preferably associated with the same sleep parameters used to modify the audio output.

Preferably, the audio output is designed to influence a sleep state of the subject. A sleep state of the subject may represent a current awake/asleep status and/or a sleep cycle (e.g. REM or non-REM sleep cycle) of the subject. Different audio outputs can influence or encourage a particular type of sleep state (i.e. encourage the sleep state of the user to change). For example, white noise may encourage a subject to fall asleep, thereby changing from an awake state to an asleep state or birdsong may encourage a subject to wake up, thereby changing from an asleep state to an awake state. This provides a more useful sleep-based system.

The set of one or more rules may comprise or consist of a machine-learning algorithm for processing the first values of the one or more sleep parameters and/or subject characteristics to determine characteristics for the audio output. A machine-learning algorithm, such as a machine-learning classifier, is a preferred example of a (complex) rule, which defines the relationship between an input and an output. Preferable embodiments utilize a machine-learning algorithm to determine suitable characteristics for the audio output. A machine-learning algorithm thereby provides a rule (or set of rules) that can be readily modified or trained to adapt to a particular subject's characteristics or response to an audio output.

Preferably, the audio output device is adapted to iteratively modify at least one characteristic of the audio output.

Thus, the second set of values can represent responses of the subject to different characteristics of the audio output. This allows the system to learn how a subject responds to particular characteristics (e.g. how quickly a sleep state of the subject changes) to thereby be able to automatically identify appropriate characteristics for audio outputs for encouraging of influencing different sleep states of the subject. For example, certain audio characteristics (e.g. loudness or type of white noise) may work better for different people in encouraging them to fall asleep. The system can thereby automatically learn and adapt to an individual subject.

The processing system may be adapted to: obtain a set of values of the one or more sleep parameters for each iterative modification to the at least one characteristics of the audio output; and modify the set of one or more rules based on the obtained set of values for each iterative modification. Each modification to the audio output can therefore be associated with a set of values for the one or more sleep parameters. This means that the specific response of the subject to different audio outputs can be isolated and used to determine which characteristics best suit the individual and/or desired sleep state for that individual.

The sleep parameter monitor is preferably adapted to monitor brain activity of the subject, so that the one or more sleep parameters comprises at least one measure of brain activity of the subject. Brain activity can be used to accurately determine a sleep state of a subject and thereby a response of the subject to a particular audio output. This provides a more accurate system. The sleep parameter monitor may, for example, comprise an electroencephalogram system for monitoring neural oscillations or "brainwaves" of the subject. Such neural oscillations are indicative of a sleep state of the subject.

Preferably, the sleep parameter monitor is adapted to monitor a brain activity in a predetermined frequency band. Neural oscillations or brain waves are an example of brain activity that can be monitored, for example, using an electroencephalogram (EEG) system. Brain activity of a subject can be divided into different bands of frequencies, where activity in different bands can represent different stages of sleep. For example, one or more EEG based derivatives may be used (in parallel) to serve as markers for the sleep stage (for instance power in Alpha, Beta, Gamma, Delta and/or Theta bands). For monitoring sleep onset, it would be particularly advantageous to monitor at least frequencies in an "alpha band" or "alpha spectrum", such as EEG power in the alpha band, commonly associated with wavelengths of between 8-15Hz, as this band of frequencies is highly responsive to a transition from an awake state to an asleep state. In one alternative (preferred over other alternatives), frequencies in the Beta band between 12.5 Hz and 30 Hz, such as EEG power in the beta band, can be monitored. Typically, brain activity (e.g. as monitored by an EEG) slows down during sleep onset and this is reflected in lowering of the power in the Beta band as the transition from wake to sleep is made.

Thus, a more accurate determination of the stage of the subject sleep can be determined and more accurate identification of response of the subject to a particular audio output can also be determined.

The sleep parameter monitor may be adapted to measure intermissions in the alpha spectrum of the monitored brain activity. As previously noted, the alpha spectrum of brain activity represents and is responsive to falling asleep, and can thereby be monitored to accurately determine response of the subject to different audio outputs. Intermissions or gaps in the alpha spectrum are particularly responsive to a subject falling asleep.

The sleep parameter monitor may comprise an electroencephalogram system adapted to obtain a raw electroencephalogram signal, and wherein the one or more sleep parameters comprises one or more parameters derived from the raw electroencephalogram signal. An electroencephalogram system provides one method of sensing neural oscillations or brain activity of the subject, whilst minimizing intrusiveness, discomfort and complexity. In particular, a power of certain frequencies or frequencies band are responsive to changes in a sleep state or sleep condition.

In some embodiments, the sleep parameter monitor is adapted to monitor certain characteristics of brain activity, such as temporal distribution of alpha waves (or any other suitable brainwaves), ripples, vertices and/or sleep spindles. Such characteristics of brain activity are representative of a particular sleep state and/or are responsive to changes in a sleep state. Identification of such characteristics may take place within predetermined frequency spectrums of brain activity.

As another example, the sleep parameter monitor may monitor a heart rate, respiration rate and/or body temperature. Each of these parameters are responsive to a change in sleep state of the subject and can therefore be considered to be sleep parameters of the subject. Thus, a sleep parameter monitor may comprise a heartrate monitor (e.g. a photoplethysmogram monitor), a breathing rate monitor and/or a thermometer. A camera is one example of a suitable heartrate and/or breathing rate monitor.

According to examples in accordance with an aspect of the invention, there is provided a method of providing audio output to a subject. The method comprises: obtaining subject characteristics and/or first values for one or more sleep parameters of the subject; processing the subject characteristics and/or first values for one or more sleep parameters of the subject using a set of one or more rules, to determine characteristics for an audio output; providing an audio output having the determined characteristics to the subject; subsequently obtaining second values for the one or more sleep parameters of the subject, said second values thereby consisting of values of the one or more sleep parameters obtained after the audio output begins being provided to the subject; and modifying the set of one or more rules based on the second values for the sleep parameters of the subject.

The step of modifying the set of one or more rules may comprise: determining the response of the one or more sleep parameters to the audio output using the second values of the one or more sleep parameters; and modifying the set of one or more rules based on the determined response of the one or more sleep parameters to the audio output.

The method may be adapted wherein: the step of providing the audio output comprises iteratively modifying at least one characteristic of the audio output; the step of obtaining second values comprises obtaining a set of values of the one or more sleep parameters for each iterative modification to the at least one characteristics of the audio output; and the step of modifying the set of one or more rules comprises modifying the set of one or more rules based on the obtained set of values for each iterative modification. In at least one embodiment, the one or more sleep parameters comprises at least one measure of brain activity of the subject.

According to examples in accordance with another aspect of the invention, there is provided a computer program comprising code means for implementing any described method when said program is run on a computer.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 shows a system for delivering an audio output to a subject;
Fig. 2 illustrates a method for delivering an audio output to a subject; and
Fig. 3 illustrates how a relative power of a band of frequencies of brain activity changes during different sleep stages.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for the purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

According to a concept of the invention, there is proposed a system and method that adapts how an audio output is generated based on a response of a subject's sleep parameters to the audio output. In particular, one or more rules used to generate the audio output are modified in response to how values of sleep parameters (i.e. parameters responsive to a sleep state of the subject) change in response to the audio output. The audio output can be iteratively modified to assess the impact of different audio outputs.

Embodiments are at least partly based on the realization that different individuals or subject, even if they share similar characteristics (e.g. age, gender etc.) respond to a same audio output in different ways, so that there is not a uniform solution to providing audio outputs for helping or assisting a subject that is (attempting to fall) asleep. It has therefore been proposed to adjust how an audio output is generated provided to the subject based on the response of sleep parameters of the subject to the audio output.

Illustrative embodiments may, for example, be employed in wearable sleep devices, e.g. headphones, or other sleep monitoring systems. Some embodiments may be formed from several devices, e.g. comprising a mobile phone, an alarm and/or an audio output system such as speakers.

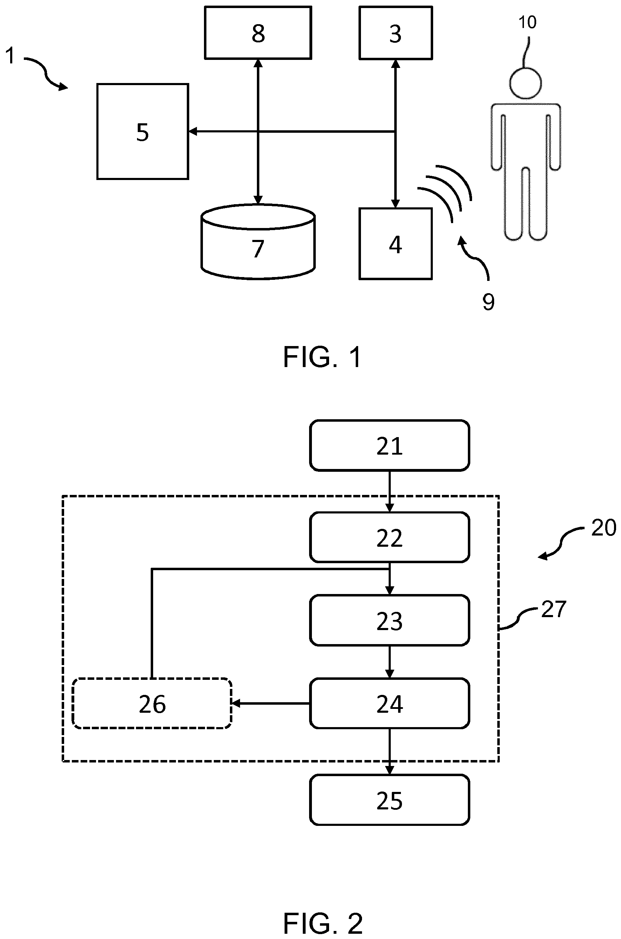

Fig. 1 illustrates a system 1 according to an embodiment of the invention. The system 1 is adapted to provide an audio output 9 to a subject 10.

The system 1 comprises a sleep parameter monitor 3 adapted to obtain values of one or more sleep parameters of a subject. The sleep parameter monitor 3 comprises any suitable sensor for obtaining values responsive to changes in a sleep state of the subject (or other desired sleep information of the subject, such as amount of slow wave activity), such as a camera (e.g. for measuring respiratory rate or subject motion), a heart rate monitor or an electroencephalogram (EEG) system formed of one or more electrodes.

The system 1 also comprises an audio output device 4. The audio output device determines characteristics for an audio output 9 and provides the audio output 9 to the subject. The audio output is preferably a suitable audio output for assisting or encouraging a change in the sleep status of the subject. Thus, the audio output may be designed to influence a sleep state of the subject. The audio system 9 may comprise a (micro)processor (not shown) for determining the characteristics and a speaker (not shown) for outputting the audio output.

An example of a suitable type of audio output (e.g. for sleep induction) includes a masking sound for masking external noise (e.g. white noise). Other examples include calming sounds, such as music, guided meditation and/or randomly generated words which all aim to reduce or modify the state of arousal of the subject. Of course, when there is a desire to wake the subject, appropriate audio outputs may also be provided (e.g. birdsong).

As another example, the audio output may comprise a sequence of sleep stimulation tones. The sleep stimulation tones may, for example, comprise a sequence of temporally separated pulses. It is known to use such pulses to increase slow wave activity (SWA) and thereby enhance deep sleep, e.g. for use during detected stages of sleep. Thus, the audio output may remain active during sleep, but the characteristics may be adjusted, such as frequency and/or amplitude depending on sleep state or other sleep parameters (i.e. based on the first values of the sleep parameters). Alternatively, they may be used only during certain sleep stages.

By way of further example, the sleep stimulation tones may comprise a sequence of 50-millisecond long tones separated from each other by a constant 1 second long inter-tone interval. Alternatively, the tones may be separated by a variable time interval, for example a random time interval. The 50ms time period is also only one possible example. The length of the inter-tone interval and/or the length of a tone may be controlled by the set of one or more rules.

In order to determine the characteristics for the audio output, the audio output device processes subject characteristics and/or (first) values of the sleep parameters monitored by the sleep parameter monitor 3 using a set of one or more rules. Thus, the audio output (or characteristics thereof) may depend upon a current status of the subject and/or metadata of the subject.

The (first) values and/or subject characteristics may be pre-processed before being processed by the set of rules. By way of example, the (first) values may be processed to determine a current sleep state of the subject, wherein the current sleep state of the subject may then be processed by the set of rules to determine the characteristics of the audio output. As another example, the age of the subject may be processed, e.g. using a classification algorithm, to determine a category of the subject (e.g. "Young", "Old"), which category can then be processed by the set of rules to determine the characteristics of the audio output. Other examples for pre-processing a set of rules would be apparent to the skilled person.

Other parameters may also be used by the set of one or more rules, such as time of day, day of week (e.g. weekday vs. weekend) and so on.

The set of one or more rules thereby defines the characteristics of the audio output based on information about the subject (e.g. their characteristics or measured values of certain sleep parameters). The set of one or more rules may therefore define, for a certain situation and/or person, one or more of the following characteristics of the audio output: a volume; a volume modulation; a frequency; a frequency modulation; a type of audio output; a waveform played; a playback speed; a number of waveforms played; a length between each of a sequence of tones; a length of each of a sequence of tones and so on. A modulation is considered to be a rise or fall of a characteristic (i.e. a change or delta of a characteristic).

Purely by way of example, the sleep parameter monitor may be adapted to determine a sleep stage of the subject, and the set of one or more rules may define audio characteristics for that sleep stage. As another example, the set of one or more rules may define audio characteristics based on an identity of the subject, so that different subjects can have different rules for generation of the audio output. Various other ways of providing an audio output based on subject information and/or monitored sleep parameters will be apparent to the skilled person.

Subject characteristics (on which the audio output could be based) include, for example: an identity of the subject; an age of the subject; a gender of the subject; a sleep schedule of the subject; calendar information of the subject; alarm information of the subject and so on.

The set of one or more rules may, for example, be stored in an electronic storage 7 of the system 1. The audio output device 4 may be adapted to retrieve the stored set of one or more rules in order to process the subject characteristics and/or first values of the one or more sleep parameters to determine the characteristics of the audio output 9. In other embodiments, the audio output device 4 itself comprises or stores the set of one or more rules.

The initial set of one or more rules (i.e. unmodified) may be selected based on characteristics of the subject (e.g. identifying a most similar subject to the present one). Otherwise, it may simply be a default set of one or more rules.

The system 1 also comprises a processing system 5. The processing system is adapted to modify the set of one or more rules based on second values of the sleep parameters (as obtained by the sleep parameter monitor 3). The second values are obtained after the audio output device 4 begins providing the audio output 9 to the subject 10.

Thus, there is a feedback system that modifies (e.g. retrains or tailors) the rule(s) on how the audio output 9 is generated. In particular, future audio outputs can be generated using a set of one or more rules that have been modified based on values of sleep parameters obtained when providing previous audio outputs.

The set of one or more rules preferably comprises or consists of a machine-learning algorithm. A machine-learning algorithm is any self-training algorithm that processes input data in order to produce output data. Here, the input data comprises subject characteristics and/or first values of the one or more sleep parameters and the output data comprises characteristics of an audio output.

Suitable machine-learning algorithms for being employed in the present invention will be apparent to the skilled person. Examples of suitable machine-learning algorithms include decision tree algorithms and artificial neural networks. Other machine-learning algorithms such as logistic regression, support vector machines or Naive Bayesian model are suitable alternatives.

The structure of an artificial neural network (or, simply, neural network) is inspired by the human brain. Neural networks are comprised of layers, each layer comprising a plurality of neurons. Each neuron comprises a mathematical operation. In particular, each neuron may comprise a different weighted combination of a single type of transformation (e.g. the same type of transformation, sigmoid etc. but with different weightings). In the process of processing input data, the mathematical operation of each neuron is performed on the input data to produce a numerical output, and the outputs of each layer in the neural network are fed into the next layer sequentially. The final layer provides the output.

Methods of training a machine-learning algorithm are well known. Typically, such methods comprise obtaining a training dataset, comprising training input data entries and corresponding training output data entries. An initialized machine-learning algorithm is applied to each input data entry to generate predicted output data entries. An error between the predicted output data entries and corresponding training output data entries is used to modify the machine-learning algorithm. This process can repeated until the error converges, and the predicted output data entries are sufficiently similar (e.g. ±1%) to the training output data entries. This is commonly known as a supervised learning technique.

For example, where the machine-learning algorithm is formed from a neural network, (weightings of) the mathematical operation of each neuron may be modified until the error converges. Known methods of modifying a neural network include gradient descent, backpropagation algorithms and so on.

The training input data entries correspond to example first values(s) of the sleep parameter and/or subject characteristics. The training output data entries correspond to characteristics of the audio output.

The processing system 5 may be adapted to retrain a machine-learning algorithm using the second set of values, i.e. the response of a subject to a particular audio output. The skilled person would be readily capable of integrating such a learning capability in a processing system. For example, if a desired response is achieved with a certain audio output, certain weightings may be upwardly revised to encourage that certain audio output for the input that achieved the desired response. If a non-desirable response is received, certain weightings may be downwardly revised to discourage that audio output.

By way of a simple example, consider a scenario in which the set of one or more rules defines characteristics of an audio output based on EEG power in the Beta band during an awake to asleep transition, where the audio output is white noise and the characteristics comprise a volume modulation. In a particular example, the set of one or more rules defines an initial time derivative of the volume (i.e. decrease of the volume over time, being a volume modulation) based on the power in the Beta band during a predetermined integration period. The desired response is a steep decrease in Beta power and the volume modulation (i.e. increase or decrease in volume) is evaluated after each integration period to determine the effect on Beta power and controls the next setting for volume modulation.

For instance if the audio output is rainfall, rain can become more or less intense (i.e. louder or quieter) as a function of measured beta power or its first derivative (i.e. increase or decrease). The speed at which the characteristics of the rainfall audio change is based on the speed of the decrease in beta power.

In another example, the set of one or more rules may comprise a rule that correlates a change in a subject's beta power (over a predetermined period of time) to a masking sound volume using a certain constant. This rule may aim to provide a masking sound that reduces the beta power, sending the subject into sleep or into a deeper sleep state. After the masking sound is played to the subject, the response of the subject's beta power (i.e. within a subsequent period of time of the same length) is monitored to determine an effect of the masking sound generated using that particular rule having that constant. In response to the subject's beta power increasing, it may be determined that the rule resulted in the masking sound volume being too low. The certain constant can then be modified so that in the next time period the rule causes the masking sound volume to stay relatively higher. In this way, the rule is adapted to a subject's specific sensitivity. In subsequent nights, the rules can then pre-emptively adjust the certain constant as a function of time, e.g. by taking the elapsed time after an "eyes closed" event into account.

Another way of personalizing or changing the set of one or more rules is to take a certain sleep parameter (e.g. beta power) and adjust the characteristics of an audio output accordingly (e.g. according to the rule, such as described above). Subsequently, the correlation of other parameters can be monitored "in the background" (i.e. not actively basing audio output on them). A periodic assessment can be made so as to correlate all of the monitored signals to the final goal (shorter sleep onset latency) and select a different governing parameter if there is one with a better correlation. The set of one or more rules may then be adapted to use additional sleep parameters (that were previously monitored, but not used to modify the audio output) to subsequently modify the audio output. For example, the sleep parameter used by the set of one or more rules could change, e.g. if a better correlation between sleep parameter and desired sleep state/benefit is identified, which thereby personalizes the set of rules.

In other words, other sleep parameters than those used to control the characteristics of the audio output may be monitored, and sleep parameters correlating with a desired sleep state or other sleep benefit (e.g. slow wave activity) can be identified as used as additional or replacement inputs for the set of one or more rules.

Such embodiments allow for increased personalization of the set of rules, because subjects/people may have different responses to changes in sleep state (e.g. stronger alpha power) so that certain sleep parameters may be more strongly measurable in some subjects than others.

Thus, there are various envisaged methods of adjusting a set of one or more rules, which include changing a relationship between a monitored sleep parameter and a characteristics of an audio output or changing which monitored sleep parameter(s) controls the characteristics of the audio output. This enables increased personalization of the audio output system.

In some embodiments, a different subset of one or more rules may be used for different purposes (i.e. depending on a desired sleep state or other sleep characteristic of the subject). For example, a first subset of one or more rules may be used when there is a desire to encourage sleep. A second, different subset of one or more rules may be used when there is a desire to encourage sleep. A third, different subset of one or more rules may be used when there is a desire to encourage certain characteristics of sleep (e.g. slow wave activity).

Other ways of implementing a set of one or more rules include IF statements, decision-trees and so on. Suitable methods of adjusting such rules would also be apparent to the skilled person.

The system preferably performs iterative adjustments to the characteristics of the audio output, and monitors the subject's response (e.g. certain sleep parameters) to the changes in the characteristics. In this way, the system can automatically learn a best relationship between monitored inputs and a desired sleep condition (as determined by the monitored subject's response). This may be used to define the set of one or more rules for controlling the characteristics of the audio output.

The system may further comprises a user interface 8. The user interface may be adapted to permit the user to manually override or modify the characteristics of the audio output provided by the audio output system (e.g. turn down a volume or change a type of audio output - such as switching between calming music and wave noises). Such manual changes may, for example, also be used by the processing system to modify or change the set of one or more rules to account for user preferences (i.e. user input may act as feedback).

While the sleep parameter monitor 3, audio output system 4 and processing system 5 (as well as the optional electronic storage 7 and user interface 8) are shown as separate entities, this is not intended to be limiting. Some and/or all of the components of system 1 and/or other components may be grouped into one or more singular devices. For example, some and/or all of the components of system 1 may be grouped as part of a headband and/or other garment(s) to be worn by the subject 10.

The audio output device is adapted to provide auditory stimuli to subject 10 prior to a sleep session, during a sleep session, after a sleep session, and/or at other times. The sounds are intended to induce, maintain, encourage and/or adjust a sleep status or sleep characteristics of a subject. The sounds may, for example, comprise a masking sound (for masking external noise), a calming sound (for soothing internal disturbances, such as stress), white noise (e.g. to mitigate tinnitus) or a sequence of temporally separated pulses (e.g. designed to increase slow wave activity).

The sleep parameter monitor 3 is used to generate output signals conveying information related to sleep-status responsive characteristics of subject 10. The detection takes place during a sleep session of subject 10, at regular intervals during a sleep session, before a sleep session, after a sleep session, and/or at other times. Thus, the sleep parameter monitor obtains values of one or more sleep parameters of a subject.

The sleep parameters are responsive to changes in a sleep state or other sleep characteristics of the subject, such as changes to: a sleep depth, a current sleep stage, slow wave activity (SWA) in subject 10, and/or other characteristics of subject 10. The monitored sleep parameters of subject 10 may be associated with rapid eye movement (REM) sleep, non-rapid eye movement (NREM) sleep, and/or other sleep. Sleep stages of subject 10 may include one or more of NREM stage N1, stage N2, or stage N3 sleep, REM sleep, and/or other sleep stages. N1 corresponds to a light sleep state and N3 corresponds to a deep sleep state. NREM stage N3 or stage N2 sleep may be slow wave (e.g., deep) sleep.

The sleep parameter monitor may comprise electroencephalogram (EEG) electrodes although other sensors may be used instead or in addition thereto. An EEG signal exhibits changes throughout a sleep session, and can therefore accurately represent a sleep parameter of the subject. In particular, a brain activity of a subject slows down during sleep, so that different frequencies of brain activity become prominent as the sleep stage of the subject changes. For example, during deep sleep, the EEG delta power is typically prominent and visible, whereas during an "awake" state, the EEG alpha or beta power is typically more prominent.

Brain activity (formed of neural oscillations) is commonly divided into a group of different frequency bands. These band include an "Alpha" band (in the region of 7.5-12.5Hz or 8-13 Hz); a "Beta" band (in the region of 12.5Hz to 30Hz); a "Gamma Band" (in the region of 30 - 100Hz or 32 - 100Hz); a "Delta" Band (in the region of 0.1-3Hz); and a Theta band (in the region of 3/4 - 7/8 Hz).

Fig. 2 illustrates a method 20 of providing audio output to a subject, e.g. to be carried out by the system described with reference to Fig. 1.

The method 20 comprises a step 21 of obtaining subject characteristics and/or first values for one or more sleep parameters of the subject.

A step 22 is then performed of processing the subject characteristics and/or first values for one or more sleep parameters of the subject using a set of one or more rules, to determine characteristics for an audio output. In step 23, this audio output having the determined characteristics is then provided to the subject.

Step 24, subsequent to step 23, comprises obtaining second values for the one or more sleep parameters of the subject. The second values therefore consist of values of the one or more sleep parameters obtained after the audio output is initially provided to the subject

Step 25 comprises modifying the set of one or more rules based on the second values for the sleep parameters of the subject.

In some embodiments, step 25 comprises pre-processing the second values to obtain a parameter derived from the second values (e.g. sleep onset time or time at which sleep state changes). Modifying the set of one or more rules may be based on the derived values.

A method of modifying the set of one or more rules has been previously described.

Steps 21 and 24 may be performed by the sleep parameter monitor, steps 22-23 can be performed by the audio output device and step 25 can be performed by the processing system of the system described with reference to Fig. 1.

Preferably, the method 20 comprises iteratively modifying at least one characteristic of the audio output. In this way, a set of values can be obtained for each modification of the at least one characteristics. In other words, each time a characteristic (or characteristics) of the audio output is changed, the response of one or more sleep parameters to the change in characteristics can be determined.

There may therefore be an additional step 26 (following step 24) of modifying the characteristics of the audio output. This forms an iterative process 27 of modifying the characteristics of the audio output 26; providing 23 the modified audio output to the subject; and obtaining 24 a set of values of the one or more sleep parameters for the modified audio output.

Accordingly, step 25 may be modified to comprise modifying the set of one or more rules based on the obtained set of values for each iterative modification. Thus, a plurality of sets of values (generated by the iterative process 27) may be used in step 25 to modify the set of one or more rules.

In preferable embodiments, each time a set of one or more values is generated, it is assessed to determine an impact of the subject's sleep. Preferably, if a predetermined number of modification have been made to the (e.g. a certain number of iterations) without improving the subject's sleep (or without achieving another desired goal, e.g. moving the subject towards an awake stage or towards a certain sleep state), the iterations are stopped, and the characteristics of the audio output are reverted back to the original settings. Iterative changes to the audio output may thereafter be stopped or paused (e.g. for the remainder of the night).

The step of assessing of the impact of the subject's sleep may depend upon the set of values generated. For example, if the set of values comprises a motion of the subject, increased motion would indicate that a subject is not falling asleep. As another example, if the set of values comprises a breathing rate, an increased breathing rate would indicate that the subject is not falling asleep. As yet another example, if the set of values comprises a power measure in a delta band (of brain activity or neural oscillations), a lack of increased delta power may indicate that the subject is not falling asleep or is not moving towards a deeper sleep state.

The period between modifications, i.e. between iterations of step 26, may be predetermined. The period may, for example, be less than 5 minutes (e.g. 1 minute), less than 10 minutes, less than 30 minutes. In other examples the period is greater (e.g. a day or more).

There is proposed an embodiment in which the monitored sleep parameter is a sleep onset time (being a time at which a subject is determined to fall asleep, e.g. based on brain activity or a threshold breathing rate), and the characteristics of the audio output are adjusted once a day (e.g. every time the subject attempts to go to sleep). The set of one or more rules (e.g. comprising a reinforcement learning algorithm or machine-learning algorithm) is used to learn what the individual optimal settings are for the subject, by making (small) adjustments to the audio output over time and measuring the effect of the audio output on the sleep onset time. The set of one or more rules can thereby determine personalized best settings for an individual and audio type.

As an example: the initial audio output could comprise white noise. White noise has equal power in each frequency. However, people experience this sound differently, for instance due to age differences in hearing ability (young people hear higher tones better) but also in how the sound experienced. The set of one or more rules could learn which power spectrum across the full frequency band is best suited for the user by adjusting the power at a certain frequency, frequency band or combination of frequency bands each time a subject attempts to go to sleep (e.g. adjust characteristics of the audio output each night) and registering the response of a sleep parameter, such as sleep onset time. Over time, the set of one or more rules can be adapted by learning which dominant frequencies correlate best with a short sleep onset and modifying the rules so that these dominant frequencies are provided to the subject.

Modifying a characteristic only once a night/day (i.e. only once every time the subject attempts to go to sleep) would result in a method that, although accurate, is slow to converge.

In an embodiment, the system may be adapted to change dynamically characteristics of the audio signal as the subject is moving, or attempting to move, from one sleep state to another (e.g. falling asleep or moving from a rapid eye movement (REM) sleep state to a non-rapid eye movement (REM) state).

In particular, the influence of adjusting audio parameters can be measured by monitoring brain activity, such as an electroencephalogram raw signal as measured by an electroencephalogram (EEG) system, during the "falling asleep" phase. The system can dynamically/iteratively change the characteristics of the audio output during the (attempted) change in sleep state and correlate said changes in the characteristics of the audio output to the changes in the EEG signal.

In particular, the system could correlate the effect of the changes to audio output with the occurrence of intermissions (i.e. gaps) in the Alpha spectrum (being the first signs of sleep onset). Thus, intermissions in the Alpha spectrum of an EEG signal may act the "one or more sleep parameters".

Alternatively, certain derivatives of the raw EEG signal can be monitored for the effect of changes in characteristics of the audio output. Typically, brain activity slows down during sleep onset and this is reflected in lowering of the power in a particular frequency band (e.g. the Beta band). Thus, changes in the power (i.e. a monitored derivative of the power) in a particular frequency band reflect a change in the sleep status of the subject (e.g. falling asleep, moving from a rapid eye movement (REM) sleep state to a non-rapid eye movement (REM) state and so on).

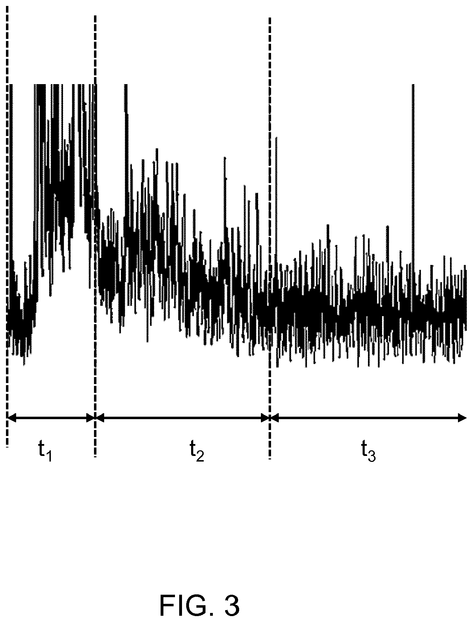

This understanding is best illustrated in Fig. 3, which illustrates a relative power in the Beta band of brain activity over time. The different time periods t₁, t₂, t₃ represent different stages of sleep. A first time period t₁ represents an "awake with eyes open" stage, a second time period t₂ represents an "awake with eyes closed" stage and a third time period t₃ represents an "asleep" stage. As illustrated in Fig. 3, the moving average of the power reduces in the different stages. In particular, the power beings reduces within the "awake with eyes closed" stage as the subject falls asleep as moves to the "asleep" stage.

Different audio output can help contribute to the movement between different stages of sleep (e.g. by masking disruptive external noises to help a subject sleep, or increasing a noise volume to help a subject wake up).

It is proposed that, whilst the subject is moving, or attempting to move, between different sleep states, the system varies audio parameters and determine the effect on the slope of an EEG signal of a particular band of frequencies. For instance, whilst the subject is in an "awake state" (but attempting to sleep, e.g. with eyes closed), the system may iteratively adjust sound intensity, frequency or modify the type of audio output. If the system records a change in the slope of the EEG signal (in that band), the adjustment is deemed to have an effect on the falling asleep process and the set of rules can then be updated with this knowledge.

Methods of updating or modifying the set of rules have previously been described, but may comprise modifying a certain constant of an algorithm forming a rule or retraining or modifying weights of a machine-learning algorithm.

In particular, the system may choose to monitor the effect of a change of an audio signal for a chosen time (for instance 1 minute, 2 minutes or 5 minutes) and compute the (change in) slope after this time. In this way the effect of changing parameters may be determined in a dynamic manner, allowing the system to learn more quickly than a once a day approach previously described.

Other suitable markers (rather than power in a particular EEG band) responsive to changes in a sleep state of a subject may be used as the one or more sleep parameters (for instance power in Alpha, Beta, Gamma, Delta and/or Theta bands, a breathing/respiration rate, a heart rate, subject motion and so on). Values derived from the aforementioned markers (e.g. sleep onset time, time of switching sleep state) may also be used as the values for the one or more sleep parameter.

In this way, the set of rules are modified based on inputs that can be adjusted (i.e. the characteristics of the audio output) and markers which are known to be indicators for a desired end result (e.g. subject falling asleep, waking up or improving sleep).

Preferably, to avoid interfering with the sleep onset time too much during experimentation (e.g. when iteratively modifying the audio output), the system may choose to limit the number of adjustments having a negative impact. Thus, if more than a certain number, e.g. no less than 3 or no less than 5, adjustments have been made that impact the sleep onset transition in a negative way, the audio output reverts back to its original settings and avoids any more changes.

Various characteristics of the audio output can be altered (e.g. during the iterative alterations) depending upon implementation details. For example, an audio device may be able to adjust the speed or volume of a particular audio output, or may be able to adjust a content of an audio output (e.g. switching between different waveforms, such as between different sounds of sea waves and ocean waves or between waveforms of slow rain and intense rain).

A system could be adapted to reward a period of particular sound when slowing down of brainwave frequency is observed (or other appropriate changes of measured parameter, such as slowing of breathing) and penalize periods of a particular sound when high or increasing speed of brain activity (or other parameter changes) is observed, by appropriately modifying the set of rules in response to the different sounds. Thus, the system can learn which sounds are associated with periods of slowing down of brain activity (falling asleep) and periods of increased brain activity (waking up). Over time the system can thereby learn how to best deal with the dynamic characteristic of the subject and adjusts itself to deliver the best sound per subject per situation.

When carrying out proposed embodiments over time, the proposed system/method can gather many sleep onset curves for e.g. increasing sleep depth, transitions from wake to deep sleep and beta power reduction. The system can learn to recognize when sleep onset issues occur and when assistance is needed. Then the system can deliver suitable sound types only when it is needed.

Thus, in some embodiments, modifying the set of one or more rules may comprise modifying the set of rules so that the audio output device only outputs audio for certain scenarios (e.g. certain sleep stages).

The invention makes use of sound generation, but this does not exclude use of other stimuli such as odors, visual stimulation, touch, taste, and/or other stimuli. For example, transcranial magnetic stimulation may be applied to subject 10 to trigger, encourage or discourage a desired sleep status.

Generally, above described examples have been described in the context of providing (audio) stimuli or audio outputs that encourage sleep, but other embodiments may aim to provide audio output for discouraging sleep or encouraging certain characteristics of sleep (e.g. production of slow wave activity). In particular, the set of rules may define characteristics for the audio output based on the subject characteristics and/or values of sleep parameters. Accordingly, the monitored sleep parameters may be responsive to any desired characteristics of sleep (e.g. measure of alertness, measure of slow wave activity and so on).

The skilled person would be readily capable of developing a processing system for carrying out a previously described method. Thus, each step of the flow chart may represent a different action performed by a processing system, and may be performed by a respective module of the processing system.

Indeed, as discussed above, embodiments make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

Examples of processing system components that may be employed in various embodiments of the present disclosure include, but are not limited to, conventional microprocessors, application specific integrated circuits (ASICs), and field-programmable gate arrays (FPGAs).

In various implementations, a processor or processing system may be associated with one or more storage media such as volatile and non-volatile computer memory such as RAM, PROM, EPROM, and EEPROM. The storage media may be encoded with one or more programs that, when executed on one or more processors and/or processing systems, perform the required functions. Various storage media may be fixed within a processor or processing system or may be transportable, such that the one or more programs stored thereon can be loaded into a processor or processing system.

It will be understood that disclosed methods are preferably computer-implemented methods. As such, there is also proposed the concept of computer program comprising code means for implementing any described method when said program is run on a computer. Thus, different portions, lines or blocks of code of a computer program according to an embodiment may be executed by a processor/computer to perform any herein described method.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

## Claims

1. A system for delivering an audio output to a subject, the system comprising:
a sleep parameter monitor adapted to obtain values of one or more sleep parameters of a subject;
an audio output device adapted to:
determine characteristics for an audio output by processing subject characteristics and/or first values of the one or more sleep parameters using a set of one or more rules;
provide the audio output having the determined characteristics to the subject; and
a processing system adapted to modify the set of one or more rules based on second values of the one or more sleep parameters, wherein said second values consist of values, of the one or more sleep parameters, obtained after the audio output device begins providing the audio output to the subject.

2. The system of claim 1, wherein the processing system is adapted to:
determine the response of the one or more sleep parameters to the audio output using the second values of the one or more sleep parameters; and
modify the set of one or more rules based on the determined response of the one or more sleep parameters to the audio output.

3. The system of claim 1 or 2, wherein the audio output is designed to influence a sleep state of the subject.

4. The system of claim 1 or 2, wherein the set of one or more rules comprises a machine-learning algorithm for processing the first values of the one or more sleep parameters and/or subject characteristics to determine characteristics for the audio output.

5. The system of any of claims 1 to 4, wherein the audio output device is adapted to iteratively modify at least one characteristic of the audio output.

6. The system of claim 5, wherein the processing system is adapted to:
obtain a set of values of the one or more sleep parameters for each iterative modification to the at least one characteristics of the audio output; and
modify the set of one or more rules based on the obtained set of values for each iterative modification.

7. The system of any of claims 1 to 6, wherein the sleep parameter monitor is adapted to monitor brain activity of the subject, so that the one or more sleep parameters comprises at least one measure of brain activity of the subject.

8. The system of claim 7, wherein the sleep parameter monitor is adapted to monitor a brain activity in a predetermined frequency band.

9. The system of claim 7 or 8, wherein the sleep parameter monitor is adapted to measure intermissions in the alpha spectrum of the monitored brain activity.

10. The system of any of claims 1 to 9, wherein the sleep parameter monitor comprises an electroencephalogram system adapted to obtain a raw electroencephalogram signal, and wherein the one or more sleep parameters comprises one or more parameters derived from the raw electroencephalogram signal.

11. A method of providing audio output to a subject, the method comprising:
obtaining subject characteristics and/or first values for one or more sleep parameters of the subject;
processing the subject characteristics and/or first values for one or more sleep parameters of the subject using a set of one or more rules, to determine characteristics for an audio output;
providing an audio output having the determined characteristics to the subject;
subsequently obtaining second values for the one or more sleep parameters of the subject, said second values thereby consisting of values of the one or more sleep parameters obtained after the audio output begins being provided to the subject; and
modifying the set of one or more rules based on the second values for the sleep parameters of the subject.

12. The method of claim 11, wherein the step of modifying the set of one or more rules comprises:
determining the response of the one or more sleep parameters to the audio output using the second values of the one or more sleep parameters; and
modifying the set of one or more rules based on the determined response of the one or more sleep parameters to the audio output.

13. The method of claim 11 or 12, wherein:
the step of providing the audio output comprises iteratively modifying at least one characteristic of the audio output;
the step of obtaining second values comprises obtaining a set of values of the one or more sleep parameters for each iterative modification to the at least one characteristics of the audio output; and
the step of modifying the set of one or more rules comprises modifying the set of one or more rules based on the obtained set of values for each iterative modification.

14. The method of any of claims 11 to 13, wherein the one or more sleep parameters comprises at least one measure of brain activity of the subject.

15. A computer program comprising code means for implementing the method of any one of claims 11 to 14 when said program is run on a computer.
